# EUROPÄISCHE PATENTSCHRIFT

(11) **EP 2 103 266 B1**
(45) Veröffentlichungstag und Bekanntmachung des Hinweises auf die Patenterteilung: **27.04.2011**
(21) Anmeldenummer: 09151477.8
(22) Anmeldetag: 28.01.2009
(51) Int. Cl.: A61B 17/435, A61D 19/04

(54) **Spüleinrichtung für eine Punktionsvorrichtung**
Flushing device for a puncturing device
Dispositif de rinçage pour un dispositif de ponction

(30) Priorität: 17.03.2008 AT 4292008
(43) Veröffentlichungstag der Anmeldung: 23.09.2009
(73) Patentinhaber: Steiner, Hans-Peter, 8010 Graz (AT)
(72) Erfinder: Steiner, Hans-Peter, 8010 Graz (AT)
(74) Vertreter: Babeluk, Michael

(56) Entgegenhaltungen:
- US-A- 4 824 434
- US-A- 5 271 379
- US-A- 5 902 279

## Beschreibung

Die Erfindung betrifft eine Spüleinrichtung für eine Punktionsvorrichtung mit einer Spülleitung und einer Punktionskanüle, mit einem Spülmittelbehälter zur Aufnahme eines Spülmittels, welcher mit der Spülleitung verbindbar ist, wobei der Spülmittelbehälter in einer Haltevorrichtung fixiert ist, die mit einer fußbetätigbaren, mechanischen Antriebsvorrichtung für die dosierte Förderung des Spülmittels verbunden ist. Die mechanische Antriebsvorrichtung weist eine Fußwippe auf, die über ein Seilzugelement oder eine flexible Antriebswelle mit der Halteeinrichtung für den als Kolbenspritze ausgeführten Spülmittelbehälter kraftschlüssig in Verbindung steht.

Im Rahmen der Sterilitätsbehandlung mit Hilfe der klassischen In-Vitro-Fertilisierung (IVF) werden die Eizellen durch Punktion des Eibläschens (Follikel) unmittelbar vor dem zu erwartenden Eisprung gewonnen. Mittels einer etwa 10 Tage dauernden Hormonkur wird die Reifung der Eizellen unterstützt, wodurch gleichzeitig mehrere Eizellen heranreifen und die Erfolgschancen der IVF erhöht werden.

Zum Teil können allerdings durch die Hormonbehandlung Schwierigkeiten auftreten, so dass für Patientinnen mit dem sogenannten PCO-Syndrom (Polyzystische Ovarien) die lebensbedrohliche Komplikation eines sogenannten ovariellen Hyperstimulationssyndroms (OHSS) auftreten kann. Eine spezielle Variante des Verfahrens, die sogenannte In-Vitro-Maturation (IVM), verhindert diese Komplikation.

Bei der IVM wird ohne vorherige Hormongabe in einem spontanen Zyklus bereits bei einer Follikelgröße von 8 mm bis 10 mm meist zwischen dem 8. und 10. Zyklustag die Follikelreifung hormonell ausgelöst, wobei nach ca. 36 bis 38 Stunden alle vorhandenen Eibläschen ab einer Größe von 3 mm bis 4 mm punktiert und unter Ultraschallkontrolle abgesaugt werden. Die Eizellen werden dann im Brutschrank 24 Stunden nachgereift und danach im Falle des Vorhandenseins eines Polkörperchens mittels der ICSI-Methode fertilisiert.

Insbesondere bei der IVM sind aufgrund der geringen Follikelgröße die meisten bekannten Punktionssets zur Gewinnung der Eizelle ungeeignet bzw. müssen spezielle Spültechniken angewandt werden um die Eizellengewinnung zu unterstützen.

Es ist bekannt Punktionsvorrichtungen mit einer Spülvorrichtung zu verwenden, wobei beispielsweise mit Hilfe eines Dreiwege-Ventils, welches an eine einlumige Punktionsnadel angeschlossen wird, je nach Stellung des Ventils gespült oder aspiriert werden kann. Dazu wird die einlumige Nadel mit einem Dreiwege-Ventil abwechselnd mit einem Spülschlauch und einer Saugeinrichtung verbunden. Nach dem Absaugen des Follikels schaltet der Reproduktionsmediziner das Ventil um, wodurch die Leitung zu einer Spülspritze frei wird. Diese wird nach den Anweisungen des Reproduktionsmediziners entweder von einer Hilfskraft händisch oder elektromotorisch aktiviert und der Follikel wieder aufgefüllt. Dreht nun der Arzt das Dreiwege-Ventil wieder in seine Ausgangsposition zurück, wird der aufgefüllte Follikel durch Aktivierung der Vakuumpumpe wieder entleert.

Ein Nachteil einlumiger Systeme ist durch das relativ große Totvolumen gegeben, welches dem Volumen der Punktionsvorrichtung von der Kanülenspitze bis zum Dreiwege-Ventil entspricht.

Es kommen auch zweilumige Punktionsnadeln zum Einsatz, deren Nachteil naturgemäß der größere Außendurchmesser von zumeist 1,6 mm (1,6 Gauge) ist, wodurch die Eizellenpunktion für die Patientin wesentlich schmerzhafter wird als mit einer einlumigen Nadel (meist 1,2 mm bzw. 1,8 Gauge).

Aus der DE 35 22 782 A1 ist in diesem Zusammenhang eine doppellumige Punktionskanüle zur Follikelpunktion bekannt geworden, welche aus einem Außenrohr mit im Wesentlichen kreisförmigen Querschnitt und einem Innenrohr besteht, dessen Außenwand in einem großen Umfangsbereich an der Innenwand des Außenrohrs anliegt, wobei das Innenrohr nur in einem kleinen Bereich mit einem Umfangswinkel kleiner 90° eine Einwölbung aufweist, welche in diesem Bereich zum Außenrohr einen Abstand aufweist und einen Spülkanal bildet. Der verbleibende Saugkanal im Innenrohr weist dadurch allerdings eine den freien Durchtritt der durch Punktion gewonnenen Eizellen behindernde Einwölbung auf. Mit Hilfe einer elektrischen Membranpumpe, an die ein steriles Schlauchsystem gekoppelt ist, wird die Follikelflüssigkeit samt Eizelle in einen Auffangbehälter gesaugt. Die Spüleinrichtung besteht hier im Wesentlichen aus einer die Spülflüssigkeit enthaltenden Kolbenspritze welche über einen Spülschlauch mit dem Spülkanal der Punktionskanüle verbunden ist.

Aus der EP 1 158 913 B1 ist eine Anordnung zum Übertragen einer Eizelle von einem Follikel bekannt, welche ebenfalls eine doppellumige Kanüle verwendet. Die Kanüle weist eine distal spitz zulaufende Punktionskanüle auf, deren proximales Ende mit einem Aufnahmebehälter für die Eizelle verbunden ist. Der Behälter für die Aufnahme der Eizelle weist eine Verbindung zu einer Vakuumquelle auf, so dass in der Punktionskanüle eine Saugwirkung erzielt werden kann. Der Spülschlauch der Vorrichtung, der einen Anschluss zu einer nicht näher bezeichneten Spülmittelquelle aufweist, ist über ein spezielles Verbindungselement am proximalen Ende der Punktionsnadel ins Innere der Punktionskanüle eingekoppelt, wo ein elastisch verformbarer Spülkanal aus einem flexiblen Material vorliegt, der bis zur Nadelspitze der Punktionsnadel reicht.

Aus der US 5,271,379 A ist eine Spüleinrichtung für eine endoskopische Einrichtung bekannt, welche in einer Halterung einen als Kolbenspritze ausgebildeten Spülmittelbehälter aufweist. Die Halterung ist mit einem Seilzugelement mit einer fußbetätigbaren, federunterstützten Wippe verbunden, wobei das distale Ende des Seils einen Adapter aufweist, der direkt am Kolben der Kolbenspritze angreift. Durch Betätigung der Wippe kann der Kolben vor- und federunterstützt zurück bewegt werden.

Aufgabe der Erfindung ist es, eine technisch möglichst einfache und kostengünstig herzustellende Spüleinrichtung für einlumige und doppellumige Punktionsvorrichtungen zu entwickeln, die sowohl bei der klassischen IVF als auch bei der In-Vitro-Maturation (IVM) eingesetzt werden kann und es dem Reproduktionsmediziner gestattet möglichst ohne Hilfspersonal aufeinanderfolgende Spül- und Saugzyklen anzuwenden. Weiters soll die Spüleinrichtung für ein 'pulsatives' Follikelspülen bei der In-Vitro-Maturation eingesetzt werden können.

Diese Aufgabe wird erfindungsgemäß dadurch gelöst, dass das Seil des Seilzugelementes mit einer verschiebbar in der Haltevorrichtung gelagerten Schubstange verbunden ist, welche ein auf den Kolben der Kolbenspritze einwirkendes Mitnehmerelement in Richtung des Kolbens der Kolbenspritze bewegt, sowie dass das Mitnehmerelement ein Sperrglied aufweist, welches bei einer Pumpbewegung der Fußwippe eine kraftschlüssige Verbindung zur Schubstange herstellt und diese bei einer Bewegung der Schubstange in die Gegenrichtung löst.

Der Arzt kann die Antriebsvorrichtung der Spüleinrichtung ohne zeitaufwändige Kommandos an sein Hilfspersonal, zeitlich exakt angepasst an die jeweiligen Erfordernisse selbst betätigen, wobei er am Ultraschall-Monitor ohne wesentliche zeitliche Verzögerung das Wiederauffüllen des Eibläschens beobachten und den Druckaufbau sowie die Füllgeschwindigkeit steuern kann. Beide Hände bleiben dabei frei für die notwendigen Manipulationen mit dem Ultraschallkopf und der Punktionsvorrichtung.

Mit Hilfe der Fußwippe kann ohne Zeitverlust sowohl der Zeitpunkt, die Menge als auch die Füllgeschwindigkeit der einzuführenden Spülflüssigkeit direkt vom Reproduktionsmediziner bestimmt werden. Eine leere Kolbenspritze kann rasch gegen eine mit Spülmittel gefüllte Spritze ausgetauscht werden, die einfach in eine Aufnahme der Haltevorrichtung eingeklickt wird.

Die Punktionsvorrichtung dient bevorzugt zur Entnahme von Eizellen für die In-Vitro-Fertilisierung (IVF)

Anstelle einer Kolbenspritze kann als Spülmittelbehälter auch ein Infusionsbeutel mit einem wesentlich größeren Fassungsvermögen für das Spülmittel in eine entsprechend angepassten Haltevorrichtung eingesetzt werden. Mit der mechanischen Antriebsvorrichtung wird bei der Betätigung der Fußwippe Druck auf den Infusionsbeutel bzw. auf geeignete Teile des Infusionsbestecks ausgeübt.

Erfindungsgemäß kann die Halteeinrichtung einen Endschalter aufweisen, der kurz vor der Endstellung des Kolbens der Kolbenspritze einen optischen oder akustischen Signalgeber betätigt. Der Arzt kann daher seine volle Aufmerksamkeit dem Ultraschall-Monitor zuwenden und wird durch ein optisches oder akustisches Signal rechtzeitig daran erinnert, eine neue mit Spülmittel gefüllte Kolbenspritze in die Halteeinrichtung einzusetzen.

Die Erfindung wird im Folgenden anhand von Zeichnungen näher erläutert. Es zeigen:
- Fig. 1: eine erfindungsgemäße Spüleinrichtung für eine doppellumige (an der Spitze einlumige) Punktionsvorrichtung für die Entnahme von Eizellen in einer zum Teil schematischen Übersichtsdarstellung;
- Fig. 2: Detail X in Fig. 1 in einer vergrößerten Schnittdarstellung;
- Fig. 3: die Spüleinrichtung gemäß Fig. 1 in einer Schnittdarstellung;
- Fig. 4: die Spüleinrichtung gemäß Fig. 1 in einer Draufsicht;
- Fig. 5: die Spüleinrichtung gemäß Fig. 1 in einer Ansicht von vorne; sowie
- Fig. 6: die erfindungsgemäße Spüleinrichtung für eine einlumige Punk- tionsvorrichtung für die Entnahme von Eizellen in einer zum Teil schematischen Übersichtsdarstellung.

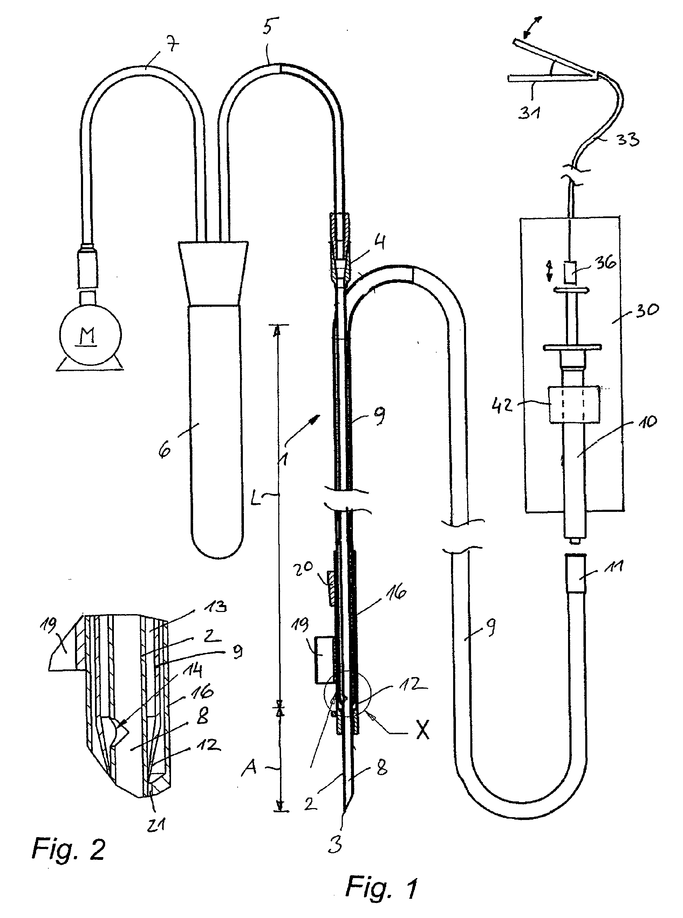

Gemäß Fig. 1 ist die Spüleinrichtung - im Wesentlichen bestehend aus dem Spülmittelbehälter 10 (z.B. eine Kolbenspritze), der Haltevorrichtung 30 für den Spülmittelbehälter und der mechanischen Antriebsvorrichtung 31 - Teil einer Punktionsvorrichtung 1 die bevorzugt für die Entnahme von Eizellen bei der In-Vitro-Maturation (IVM) eingesetzt wird. Die Punktionsvorrichtung 1 weist eine Punktionskanüle 2 mit einer geschliffenen Nadelspitze 3 am distalen Ende auf, wobei an deren proximalen Ende 4 über einen Verbindungsschlauch 5 ein Aufnahmebehälter 6 für die Eizelle bzw. für das den Eizell-Kumulus-Komplex enthaltende Punktat angeschlossen werden kann. Der Aufnahmebehälter 6 weist eine Anschlussleitung 7 zu einer Vakuumquelle; beispielsweise einer elektrisch betriebenen Membranpumpe M, auf, um im Lumen der als Saugkanal 8 ausgeführten Punktionskanüle 2 den für die Probengewinnung nötigen Unterdruck erzeugen zu können.

Weiters ist eine als flexibler Spülschlauch ausgeführte Spülleitung 9 vorgesehen, mit welcher ein Spülmittel aus dem am proximalen Ende 11 befestigbaren Spülmittelbehälter 10 der Haltevorrichtung 30 eingebracht werden kann. Der Spülmittelbehälter 10 ist hier als Kolbenspritze ausgeführt, welche über einen Luer-Anschluss am proximalen Ende 11 der Spülleitung 9 angesteckt werden kann. Der in der Haltevorrichtung 30 fixierte Spülmittelbehälter 10 ist mit einer fußbetätigbaren, mechanischen Antriebsvorrichtung 31 für die dosierte Förderung des Spülmittels verbunden. Durch eine gleichzeitige, aufeinander abgestimmte Saug-und Spültätigkeit der Membranpumpe M und der Kolbenspritze 10 kann ein optimales Ergebnis bei der Follikelspülung erzielt werden.

Die Spülleitung 9 umfasst die Punktionskanüle 2 und ist an einer von der Nadelspitze 3 ca. 6 cm bis 9 cm beabstandeten Verbindungsstelle 12 flüssigkeitsdicht an dieser befestigt. Es entsteht so zwischen der Außenwand der Punktionskanüle 2 und der Innenwand der Spülleitung 9 ein Spülkanal 13, welcher an dessen distalem Ende zumindest eine Übertrittsöffnung 14 in den Saugkanal 8 aufweist (siehe Fig. 2). Dadurch entsteht im Anschluss an die Nadelspitze 3 ein dünner, einlumiger Punktionsbereich A, welcher sich hervorragend für die Eizellengewinnung eignet.

Mit Hilfe der erfindungsgemäßen Spüleinrichtung können die Strömungsverhältnisse im einlumigen Punktionsbereich A der Nadel ohne Verwendung eines Dreiwege-Ventils sehr rasch umgekehrt werden kann. Diese Konstruktion ermöglicht es dem Arzt während des Anliegens eines Aspirationsdruckes von 80 mm/Hg an der Saugleitung 5 mittels der Kolbenspritze 10, mehrmals nacheinander den Aspirationsdruck zu überwinden und somit den Follikel pulsativ binnen weniger Sekunden mehrfach, beispielsweise 3- bis 4-mal, zu spülen.

Die Spülleitung 9 besteht beispielsweise aus einem flexiblen Schlauch, welcher an der flüssigkeitsdichten Verbindungsstelle 12 eine Durchmesserverengung, eine Schrumpfschlauchverbindung oder eine Klebeverbindung aufweist.

Der einlumige Punktionsbereich A anschließend an die Nadelspitze 3 beträgt je nach Anwendungsgebiet beispielsweise 10% bis 25% der Länge L des doppellumigen Bereichs der Punktionskanüle 2, wobei der Außendurchmesser der Punktionskanüle 2 beispielsweise 0,8 mm bis 1,2 mm beträgt.

Die Punktionsvorrichtung 1 weist im doppellumigen Bereich einen Führungsadapter 16 für einen (hier nicht dargestellten) Ultraschallkopf auf, der auf die Spülleitung bzw. auf den flexiblen Spülschlauch 9 aufsteckbar ist, bis zur Verbindungsstelle 12 reicht und den einlumigen Punktionsbereich A frei lässt. Der Führungsadapter 16 besteht im Wesentlichen aus einer rohrförmigen Aufnahme mit einem Klemmelement 19 zur Befestigung des Führungsadapters 16 am Ultraschallkopf. Am distalen Ende weist der Adapter 16 eine Öffnung 21 auf, durch die die Punktionskanüle austritt und zentriert wird (siehe Fig. 2).

Weiters weist der Führungsadapter 16 eine keilförmige Auflage 20 auf, mit welcher ein Winkel von ca. 3° bis 10° zwischen der Längsachse des Führungsadapters 16 und der Längsachse des Ultraschallkopfes einstellbar ist.

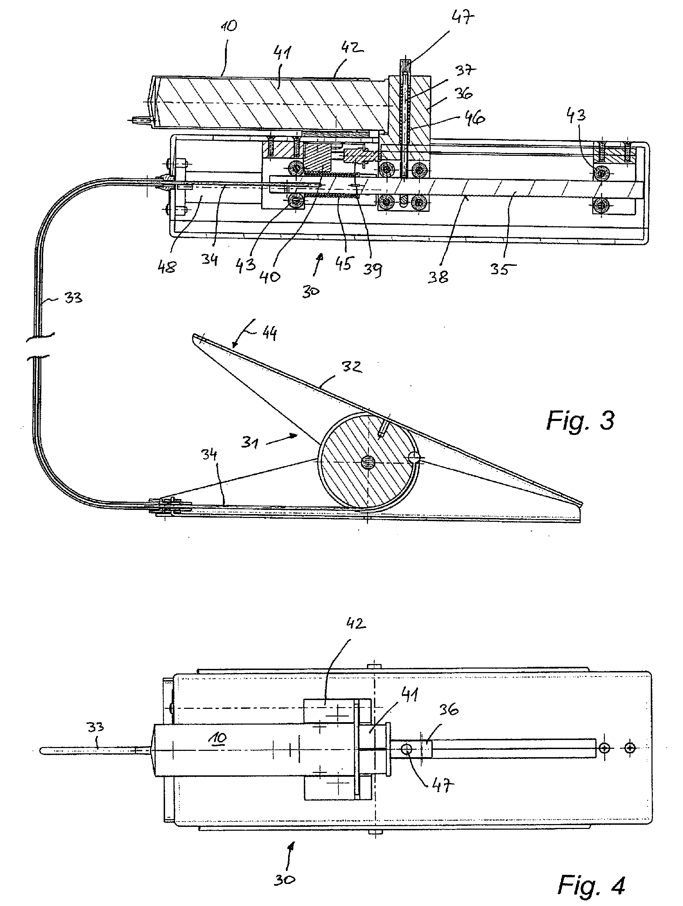

Wie im Detail in den Fig. 3 bis Fig. 5 dargestellt ist, weist die mechanische Antriebsvorrichtung 31 eine Fußwippe 32 auf, die über ein Seilzugelement 33 (oder eine flexible Antriebswelle) mit der Halteeinrichtung 30 für den als Kolbenspritze ausgeführten Spülmittelbehälter 10 kraftschlüssig in Verbindung steht. Die Kolbenspritze kann in die Aufnahme 42 der Haltevorrichtung eingeklickt werden und ist dadurch einfach auszutauschen.

Das Seil 34 des Seilzugelementes 33 ist mit einer verschiebbar in der Haltevorrichtung 30 mittels Lagerelementen 43 gelagerten Schubstange 35 verbunden, welche ein auf den Kolben 41 der Kolbenspritze 10 einwirkendes Mitnehmerelement 36 in Richtung des Kolbens 41 der Kolbenspritze 10 bewegt.

Das Mitnehmerelement 36 weist ein Sperrglied 37 auf, welches bei einer Pumpbewegung der Fußwippe 32 in Richtung des Pfeils 44 eine kraftschlüssige Verbindung zur Schubstange 35 herstellt und diese bei einer durch eine Feder 45 bewirkte Bewegung der Schubstange 35 in die Gegenrichtung löst. Das Sperrglied 37 wird von einer Feder 46 belastet und greift nach Art einer Sperrklinke in eine Verzahnung 38 an der Schubstange 35 ein. Weiters ist das Sperrglied 37 von Hand lösbar (mittels Fingerdruck auf den aus dem Mitnehmerelement 36 ragenden Teil 47), wonach das Mitnehmerelement 36 auf der Schubstange 35 nach hinten verschoben werden kann, um die Kolbenspritze 10 gegen eine neue, mit Spülmittel gefüllte auszutauschen.

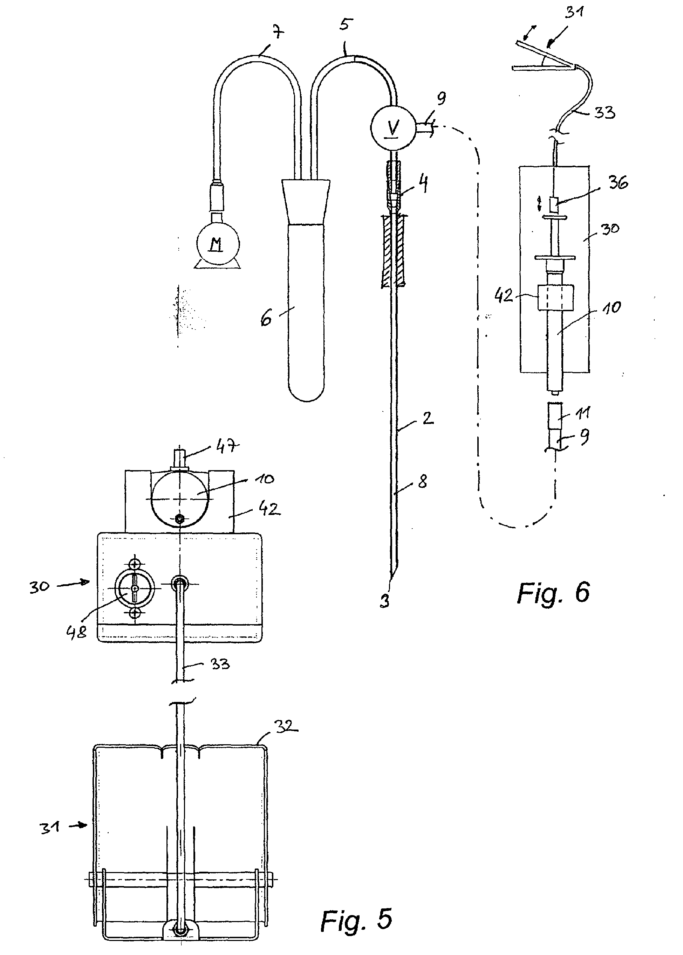

Weiters kann die Halteeinrichtung 30 einen Endschalter 39 aufweisen, der kurz vor der Endstellung des Kolbens 41 der Kolbenspritze 10 einen optischen oder akustischen Signalgeber 40 betätigt, um den Reproduktionsmediziner auf den zur Neige gehenden Füllstand des Spülmittels hinzuweisen. Mit 48 ist in Fig. 5 ein Batteriefach in der Halteeinrichtung 30 bezeichnet, welches eine Energiequelle für den Signalgeber 40 aufnimmt.

In Fig. 6 ist die erfindungsgemäße Spüleinrichtung im Zusammenhang mit einer einlumigen Punktionsvorrichtung dargestellt, deren Punktionskanüle 2 mit Hilfe eines Drei-Wege-Ventils V mit einer Vakuumquelle, z.B. mit der Membranpumpe M oder mit der Spüleinrichtung 30, 31 verbunden werden kann.

Bei der herkömmlichen IVF, bei der nach der Hormonbehandlung Follikel bis zu einer Größe von 2,5 cm abgesaugt werden, fixiert der Arzt an das proximale Ende 4 der Punktionskanüle 2 das Drei-Wege-Ventil V. Dieses wird durch einen Schlauch 5 mit einer elektronischen Vakuumpumpe M verbunden. Dazwischen geschaltet ist ein Aufnahmebehälter 6, welcher das Punktat auffängt. Die Spülleitung 9 führt zur Kolbenspritze 10 (z.B. eine 60 ml -Spritze), die in die Haltevorrichtung 30 der Spüleinrichtung eingeklickt ist.

Die mechanische Antriebsvorrichtung 31 der Spüleinrichtung wird beispielsweise durch den linken Fuß des Arztes aktiviert. Mit dem rechten Fuß aktiviert er die Membranpumpe M. Erscheint im Ultraschall der Follikel als leer, kann der Arzt mit einer Hand das Drei-Wege-Ventil umschalten, so dass der Weg zur Spüleinrichtung 30, 31 frei wird. Mit der anderen Hand hält er die Ultraschallsonde samt Nadeladapter (hier nicht dargestellt). Durch die Aktivierung der mechanischen Spüleinrichtung wird der Follikel mit Spülmedium gefüllt, und kann durch Manipulation des Drei-Wege-Ventils V wiederholt abgesaugt und gespült werden.

## Patentansprüche

1. Spüleinrichtung für eine Punktionsvorrichtung (1) mit einer Spülleitung (9) und einer Punktionskanüle (2), mit einem Spülmittelbehälter (10) zur Aufnahme eines Spülmittels, welcher mit der Spülleitung (9) verbindbar ist, wobei der Spülmittelbehälter (10) in einer Haltevorrichtung (30) fixiert ist, die mit einer fußbetätigbaren, mechanischen Antriebsvorrichtung (31) für die dosierte Förderung des Spülmittels verbunden ist und wobei die mechanische Antriebsvorrichtung (31) eine Fußwippe (32) aufweist, die über ein Seilzugelement (33) oder eine flexible Antriebswelle mit der Halteeinrichtung (30) für den als Kolbenspritze ausgeführten Spülmittelbehälter (10) kraftschlüssig in Verbindung steht, **dadurch gekennzeichnet, dass** das Seil (34) des Seilzugelementes (33) mit einer verschiebbar in der Haltevorrichtung (30) gelagerten Schubstange (35) verbunden ist, welche ein auf den Kolben der Kolbenspritze (10) einwirkendes Mitnehmerelement (36) in Richtung des Kolbens der Kolbenspritze (10) bewegt, sowie dass das Mitnehmerelement (36) ein Sperrglied (37) aufweist, welches bei einer Pumpbewegung der Fußwippe (32) eine kraftschlüssige Verbindung zur Schubstange (35) herstellt und diese bei einer Bewegung der Schubstange (35) in die Gegenrichtung löst.

2. Spüleinrichtung nach Anspruch 1, **dadurch gekennzeichnet, dass** die Punktionsvorrichtung (1) zur Entnahme von Eizellen für die In-Vitro-Fertilisierung (IVF) dient.

3. Spüleinrichtung nach Anspruch 1 oder 2, **dadurch gekennzeichnet, dass** das Sperrglied (37) federbelastet und von Hand lösbar ist und in eine Verzahnung (38) an der Schubstange (35) nach Art einer Sperrklinke eingreift.

4. Spüleinrichtung nach einem der Ansprüche 1 bis 3, **dadurch gekennzeichnet, dass** die Halteeinrichtung (30) einen Endschalter (39) aufweist, der kurz vor der Endstellung des Kolbens der Kolbenspritze (10) einen optischen oder akustischen Signalgeber (40) betätigt.

## Claims

1. Flushing device for a puncturing apparatus (1) with a flushing line (9) and a puncturing cannula (2), comprising a medium container (10) for the flushing medium, which can be connected to the flushing line (9), said medium container being held in a holding means (30), which in turn is connected to a pedal-operated mechanical drive device (31) for metered feeding of the flushing medium, the mechanical drive device (31) being provided with a hinged pedal (32), which is force-connected via a rope element (33) or a flexible drive shaft to the medium container (10) configured as a piston syringe, **characterised in that** the rope (34) of the rope element (33) is connected to a push-rod (35) which is slidably borne in the holding means (30), said push-rod (34) moving an activator element (36) acting on the piston of the piston syringe (10) in the direction towards said piston of the piston syringe (10), and that the activator element (36) has a locking member (37), which will establish a force-locked connection with the push-rod (35) upon a pumping movement of the hinged pedal (32) and will release this connection if the push-rod (35) moves in the reverse direction.

2. Flushing device according to claim 1, **characterised in that** the puncturing apparatus (1) serves for the taking of egg-cells for in-vitro fertilization (IVF).

3. Flushing device according to claim 1 or 2, **characterised in that** the locking member (37) is spring-loaded and manually releasable and engages a toothed section (38) of the push-rod (35) like a pawl.

4. Flushing device according to any of claims 1 to 3, **characterised in that** the holding means (30) is provided with a terminal switch (39), which activates an optical or acoustical signal source (40) just before the piston of the piston syringe (10) reaches its terminal position.

## Revendications

1. Installation de rinçage d'un dispositif de ponction (1) comportant une conduite de rinçage (9) et une canule de ponction (2), un réservoir d'agent de rinçage (10) pour recevoir un agent de rinçage et qui peut être relié à la conduite de rinçage (9),
- le réservoir d'agent de rinçage (10) étant fixé dans un dispositif de support (30) relié à un dispositif d'entraînement (31), mécanique commandé par le pied pour assurer un transfert dosé d'agent de rinçage, et
- le dispositif d'entraînement mécanique (31) comporte une pédale (32) reliée par une liaison par la force, par un élément de câble de traction (33) ou par un arbre d'entraînement souple à l'installation de support (30) pour le réservoir d'agent de rinçage (10) réalisé comme une seringue à piston,
installation **caractérisée en ce que**
le câble (34) de l'élément de traction (33) est relié à une tige de poussée (35) coulissant dans le dispositif de support (30), cette tige déplaçant un élément d'entraînement (36) agissant sur le piston de la seringue à piston (10) dans le sens du piston de la seringue (10), et
l'élément d'entraînement (36) comporte un organe de blocage (37) qui, lors du mouvement de pompage de la pédale (32), réalise une liaison par la force avec la tige de poussée (35) et libère celle-ci pour un mouvement de la tige de poussée (35) dans la direction opposée.

2. Installation de rinçage selon la revendication 1,
**caractérisée en ce que**
le dispositif de ponction (1) sert à prélever des cellules d'ovaires pour la fertilisation in-vitro (FIV).

3. Installation de rinçage selon la revendication 1 ou 2,
**caractérisée en ce que**
l'organe de blocage (37) est chargé par un ressort et peut se libérer à la main et une denture (38) pénètre dans la tige de poussée (35) à la manière d'un verrou.

4. Installation de rinçage selon l'une des revendications 1 à 3, **caractérisée en ce que**
l'installation de support (30) comporte un commutateur de fin de course (39) qui actionne un générateur de signal optique ou acoustique (40) juste avant la position de fan de course du piston de la seringue à piston (10).
